(19) Europäisches Patentamt
European Patent Office
Office européen des brevets

(11) Veröffentlichungsnummer : **0 071 903**
**B1**

(12) # EUROPÄISCHE PATENTSCHRIFT

(45) Veröffentlichungstag der Patentschrift : **28.08.85**

(51) Int. Cl.⁴ : **C 07 D475/00**, C 07 D475/14, A 61 K 31/505

(21) Anmeldenummer : **82106888.9**

(22) Anmeldetag : **30.07.82**

(54) **Neue Diglycidyl-pteridin-Verbindungen, Verfahren zu ihrer Herstellung und ihre Anwendung in Arzneimitteln mit cytostatischer Wirkung.**

(30) Priorität : **07.08.81 DE 3131396**

(43) Veröffentlichungstag der Anmeldung : **16.02.83 Patentblatt 83/07**

(45) Bekanntmachung des Hinweises auf die Patenterteilung : **28.08.85 Patentblatt 85/35**

(84) Benannte Vertragsstaaten : **AT BE CH DE FR GB IT LI NL**

(56) Entgegenhaltungen :
**EP-A- 0 033 503**
**DE-A- 2 815 182**
**DE-A- 2 953 309**
**US-A- 4 183 934**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Möller, Hinrich, Dr.**
**Schumannstrasse 11**
**D-4019 Monheim (DE)**
Erfinder : **Hase, geb. Kornrumpf, Brigitte, Dr.**
**Millrather Weg 29**
**D-4006 Erkrath 1 (DE)**
Erfinder : **Konrad, Günther, Dr.**
**Feuerbachweg 12**
**D-4010 Hilden (DE)**
Erfinder : **Wilk, Hans-Christoph, Dr.**
**An der Obererft 94f**
**D-4040 Neuss (DE)**
Erfinder : **Zeidler, Ulrich, Dr.**
**Heinrich-Lersch-Strasse 19**
**D-4000 Düsseldorf 13 (DE)**

**EP 0 071 903 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der DE-OS 29 07 349 sind Arzneimittelzubereitungen mit cytostatischer Wirksamkeit, die als pharmakologischen Wirkstoff Triglycidylisocyanurat enthalten. In der EP-A-33 503 werden in Verallgemeinerung dieses Wirkstoffprinzips Arzneimittelzubereitungen mit cytostatischer Wirksamkeit beschrieben, die gekennzeichnet sind durch einen Gehalt an N-Heterocyclen, die wenigstens 2 Glycidyl-substituierte N-Atome in Amid- und/oder Imidform in das Ringsystem eingebunden, enthalten. Im einzelnen offenbart die Europäische Patentanmeldung eine Mehrzahl von speziellen Stoffklassen der genannten Art, die sich in Arzneimittelzubereitungen mit tumorhemmender Wirkung einsetzen lassen.

Gegenstand der vorliegenden Erfindung sind Diglycidylsubstituierte Pteridin-Verbindungen, die als solche bisher nicht bekannt sind und sich ebenfalls durch eine hohe Wirksamkeit bei ihrer Verwendung in Arzneimittelzubereitungen mit cytostatischer Aktivität auszeichnen.

Gegenstand der Erfindung sind in einer ersten Ausführungsform die neuen Diglycidyl-pteridin-Verbindungen der im folgenden angegebenen allgemeinen Formel I. Die Erfindung betrifft weiterhin das Verfahren zur Herstellung dieser neuen Diglycidyl-pteridin-Verbindungen sowie ihre Anwendung in pharmakologischen Zubereitungen mit cytostatischer Wirkung.

Gegenstand der Erfindung sind dementsprechend in einer ersten Ausführungsform neue Glycidyl-substituierte pteridin-Verbindungen der allgemeinen Formel I

(I)

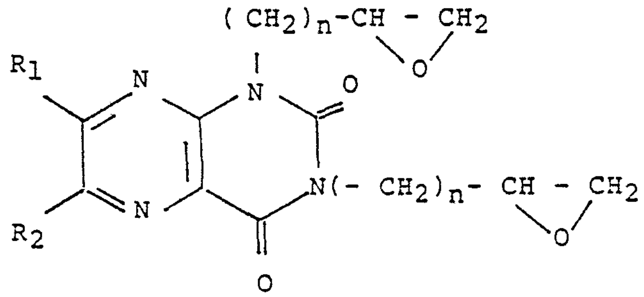

in der $R_1$ und $R_2$ gleich oder verschieden sind und Wasserstoff oder Kohlenwasserstoffreste mit bis zu 12 C-Atomen, die auch N, O, S und/oder P als Heteroatome enthalten können, bedeuten, wobei $R_1$ und $R_2$ auch gemeinsam zu einem carbocyclischen oder heterocyclischen Ring geschlossen sein können, der gewünschtenfalls einen Halogen-, Alkoxy-, Cyano-, Hydroxyl-, N-substituierten Amino-, Alkylsulfoxy-, Arylsulfoxy-, Aroxy- oder Acyloxysubstituenten enthalten kann. Dabei hat n den Zahlenwert von 1 oder 2, vorzugsweise von 1.

Das erfindungsgemäße Verfahren zur Herstellung dieser neuen Diglycidyl-pteridin-Verbindungen ist dadurch gekennzeichnet, daß man die 1,3-N-unsubstituierten Ausgangsverbindungen der allgemeinen Formel II

(II)

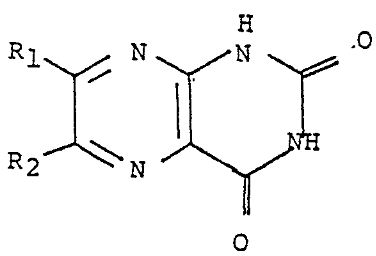

in an sich bekannter Weise mit Epihalohydrinin unsetzt und nachfolgende Halogenwasserstoff mittels Alkali abspaltet. Die neuen Glycidyl-substituierten Pteridin-Verbindungen der allgemeinen Formel I liegen in ihrer Zubereitungsform als Arzneimittel gewöhnlich in Abmischung mit üblichen Hilfsbeziehungsweise Trägerstoffen für pharmakologische Zubereitungen vor.

Der Wirkungsmechanismus der im Rahmen der Erfindung eingesetzten Verbindungen ist im einzelnen nicht geklärt. Vermutlich sind aber auch hier die vorliegenden zwei Glycidyl-Gruppen für die cytostatische Wirkung von herausragender bedeutung. Alle erfindungsgemäß beschriebenen Verbindungen der allgemeinen Formel I kennzeichnen sich durch das Vorliegen der zwei Glycidyl-Gruppen. Daneden liegen die variierbaren Reste $R_1$ und $R_2$ vor. Es ist möglich, daß über diese Reste Einfluß auf die Verteilung von liphophilen und hydrophilen Präferenzen genommen wird und daß damit in gewissem Aussmaß die Aufnahme der Verbindungen durch den Organismus gesteuert werden kann.

Die Reste $R_1$ und $R_2$ sind gemäß der vorher gegebenen Definition Wasserstoff oder Kohlenwasserstoffreste mit bis zu 12 C-Atomen. Sie können dabei gleich oder verschieden sein. Der Bergiff Kohlenwasserstoffreste umfaßt auch entsprechende Reste mit Heteroatomen. Als Heteroatome kommen insbesondere N,O,S und/oder P in Betracht. Bevorzugt enthält jeder dieser Reste nicht mehr als 8 Kohlenstoffatome. Interessant können insbesondere Reste sein, die bis zu 6 oder vorzugsweise sogar nur

bis zu 4 Kohlenstoffatome enthalten, wobei diese Zahlenwerte unabhängig von der jeweiligen Struktur zu verstehen sind und sich lediglich auf die Summe aller Kohlenstoffatome im betroffenen Rest beziehen.

Bedeutet $R_1$ und/oder $R_2$ einen Aryl-, Aralkyl, Alkarylrest, so sind hier insbesondere 1-kernige Substituenten bevorzugt. Typische Vertreter sind Phenyl, Benzyl, Tolyl, Xylyl und verwandte Verbindungen. Auch bei den cycloaliphatischen Ringen für diese Reste $R_1$ und/oder $R_2$ sind einkernige Ringsysteme auf der Basis von Cyclopentyl, Cyclohexyl und ihren Abkömmlingen bevorzugt. Entsprechende heterocyclische Reste, insbesondere also 1-kernige Ringverbindungen mit O,N und/oder S im System fallen in den Rahmen der Erfindung. Die Ringsysteme können dabei bevorzugt 1,2 oder 3 solcher Heteroatome enthalten. Diese heterocyclischen Reste enthalten vorzugsweise 5 oder 6 Ringglieder. Gewünschtenfalls können alle hier genannten ringförmingen Substituenten, seien sie aromatischer oder cycloaliphatischer Natur, ihrerseits weitere Substituenten aufweisen. Geeignete Substituenten sind beispielsweise Halogen, Hydroxyl oder Alkoxy.

Weichen $R_1$ und/oder $R_2$ in ihrer Bedeutung von Wasserstoff ab, dann bedeuten sie in einer bevorzugten Ausführungsform der Erfindung gleiche oder verschiedene und gegebenenfalls substituierte Alkylreste. Diese Alkylreste können geradkettig oder verzweigt und gesättigt oder ungesättigt sein und enthalten vorzugsweise nicht mehr als 6 insbesondere nicht mehr als 4 Kohlenstoffatome. Als Substituenten kommen beispielsweise Halogen, Alkoxy oder Cyano in Betracht. Die Erfindung ist jedoch nicht auf diese bestimmten Substituenten eingeschränkt, andere mögliche Substituenten sind beispielsweise Hydroxyl, N-substituiertes Amino, Alkylsulfoxy, Arylsulfoxy, Aroxy und/oder Acyloxy, wobei der Substituent auch heterocyclischer Natur sein kann. Hierbei können dann derart substituierte Reste ein- oder mehrfach mit den genannten Gruppen substituiert sein.

Bevorzugt liegen 1 bis 3 der genannten Substituenten am jeweiligen Rest $R_1$ beziehungsweise $R_2$ vor. Liegen an einem solchen substituierten Rest Gruppen vor, die ihrerseits Kohlenstoffwasserreste enthalten, so weisen diese substituierenden Gruppen vorzugsweise nicht mehr als 8 insbesondere nicht mehr als 6 C-Atome auf.

Die Reste $R_1$ und $R_2$ können ihrerseits zu einem carbocyclischen oder heterocyclischen Ringsystem geschlossen sein. Dieses Ringsystem besitzt vorzugsweise 5 oder 6 Ringglieder und kann gewünschtenfalls mit einem der genannten Substituenten substituiert sein. Auch hier sind als bevorzugte Heteroatome N, O, S und/oder P zu nennen.

In einer weiterhin bevorzugten Ausführungsform weisen die Substituenten $R_1$ und $R_2$ in der Summe ihrer Kohlenstoffatome nicht mehr als 12 vorzugsweise nicht mehr als 10 Kohlenstoffatome auf. Diese Zahlen gelten insbesondere einschließlich eventueller weiterer Substituenten an diesen Resten $R_1$ und/oder $R_2$. Insbesondere beträgt die Summe der Kohlenstoffatome der Reste $R_1$ und $R_2$ zusammen nicht mehr als 6 und insbesondere nicht mehr als 4 C.-Atome.

Die Herstellung von Wirkstoffen der allgemeinen Formel I ist ein weiterer Gegenstand der Erfindung. Grundsätzlich kann sie in an sich bekannter Weise erfolgen, dabei seien die folgenden Möglichkeiten aufgezählt :

1. Ein erster Verfahrensweg beruht auf der Umsetzung der in 1,3-Stellung unsubstituierten Pteridin-Verbindungen der allgemeinen Formel II

(II)

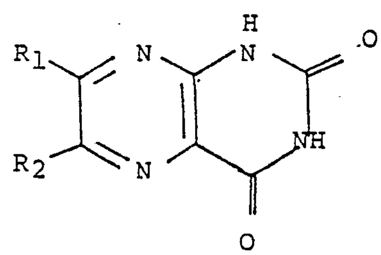

in der $R_1$ und $R_2$ die angegebene Bedeutung haben mit Epihalohydrinen. Die Herstellung dieser Ausgangsverbindungen der allgemeinen Formel II kann nach literaturbekannten Verfahren erfolgen. Nachfolgend wird Halogenwasserstoff mittels Alkali abgespalten.

Als Epihalohydrin-Verbindung wird vorzugsweise Epichlorhydrin eingesetzt. Die Umsetzung erfolgt in an sich bekannter Weise. Sie kann in Gegenwart einer geringen Menge einer basischen Komponente oder einer quartären Ammoniumverbindung als Katalysator durchgeführt werden (vergleiche hierzu beispielsweise Houben-Weyl « Methoden der organischen Chemie » Band 14/2 (1963, 497, 547). Als Alkali wird vorzugsweise NaOH in Pulver-Form oder als konzentrierte wäßrige Lösung verwendet.

2. In einer Modifikation dieses Weges wird die Ausgangsverbindung der allgemeinen Formel II nicht unmittelbar mit der Epoxidverbindung umgesetzt. Stattdessen erfolgt zunächst ihre Umsetzung mit Allylhalogeniden, in denen an Stelle der Epoxidgruppe eine olefinische Doppelbindung vorliegt, woraufhin die entstandenen Diallyl-substituierten Pteridin-Verbindungen epoxidiert werden.

Die Epoxidation kann in an sich bekannter Weise mit Persäure vorgenommen werden. Die Umsetzung von Allylhalogeniden mit einer strukturanalogen Verbindung, nämlich mit Cyanursäure ist beispielsweise beschrieben in der US-PS 3 376 301. Die Epoxidation von Allylisocyanuraten mit Persäuren ist zum Beispiel in Houben-Weyl aaO, Band 6/3 385 ff. beschrieben. Sie kann zum Beispiel in

Gegenwart einer geringen Menge einer quartären Ammoniumverbindung als Katalysator durchgeführt werden. Analog werden diese Verfahrensmöglichkeiten auf die Herstellung der erfindungsgemäß geschilderten neuen Pteridin-Verbindungen der allgemeinen Formel I angewandt.

Die Umsetzung der Pteridin-Ausgangsverbindungen mit Epihalohydrinen beziehungsweise Allylhalogeniden erfolgt zweckmäßigerweise im Temperaturbereich von etwa 50 bis 150 °C, vorzugsweise von etwa 70 bis 125 °C. Allylhalogenid- beziehungsweise Epihalohydrin wird wenigstens in den erforderlichen Molverhältnissen eingesetzt, es kann aber auch mit beträchtlichem Überschuß gearbeitet werden, beispielsweise mit Molverhältnissen bis zu 10 : 1. Das Arbeiten mit Molverhältnissen im Bereich von 2 bis 4 Mol Allylhalogenid beziehungsweise Epihalohydrin je Mol Pteridin-Ausgangsverbindung kann besonders zweckmäßig sein. Die bevorzugten Allylhalogenide beziehungsweise Epihalohydrine enthalten Chlor oder gegebenenfalls Brom als Halogen.

Die Reaktion kann in polaren, insbesondere aprotischen Lösungsmitteln vorgenommen werden, die wenigstens einen der Reaktionspartner teilweise lösen und den Reaktanten gegenüber nicht reaktiv sind. Die bevorzugte Reaktionszeit beträgt 1 bis 10 Stunden, insbesondere 2 bis 5 Stunden.

Auch die Epoxidation der Allylgruppierung mittels Persäure wird vorzugsweise in Lösungsmitteln durchgeführt. Geeignet sind polare Lösungsmittel beispielsweise Halogenkohlenwasserstoffe oder Alkohole. Die geeignete Reaktionstemperatur liegt üblicherweise im Bereich von 0 bis 50 °C, insbesondere zwischen etwa 10 und 30 °C. Die Persäure wird zweckmäßigerweise in annähernd äquivalenter Menge oder nur in leichtem Überschuß eingesetzt. m-Chlorperbenzoesäure ist als Handelsprodukt leicht zugänglich and für die Durchführung der Reaktion geeignet. Die Reaktionsdauer liegt in der Regel im Bereich von 24 Stunden oder mehr, beispielsweise bis zu 48 Stunden.

Die Verbindungen der allgemeinen Formel I in gereinigter und in Substanz gewonnener Form fallen als solche in den Rahmen der Erfindung. Insbesondere handelt es sich hierbei um Verbindungen der entsprechenden Konstitution in solcher Form insbesondere solcher Reinheit, die ihre Verwendung als Arzneimittel zulässt.

Gegenstand der Erfindung ist schließlich die Verwendung der Verbindungen der allgemeinen Formel I zur Therapie maligner Neoplasien. Einzelgaben der Verbindungen in Höhe von 1 bis 200 mg/Tag können zweckmäßig sein. Zur Verwendung als Cancerostatica sollten die Wirksubstanzen mittels geeigneter Vehikel appliziert werden. Hier eignen sich die üblichen Hilfs- beziehungsweise Trägerstoffe für pharmakologische Zubereitungen. Die erfindungsgemäß eingesetzten Verbindungen sind wirksam gegen verschiedene Leukämieformen wie maligne Neoplasmen, wie Lungenkarzinom, Colonkarzinom, Melanome, Ependymoplastom und Sarkome. Eine Kombinationstherapie in Verbindung mit anderen Cytostatika ist möglich.

Ganz allgemein gilt für die im Rahmen der Erfindung eingesetzten Verbindungen der allgemeinen Formel I mit ihren Resten $R_1$ und $R_2$, daß diese wenigstens unter Normalbedingungen keine oder keine wesentliche Reaktivität mit den Epoxidgruppen der Glycidylsubstituenten zeigen. Auf diese Weise ist sichergestellt, daß die erfindungsgemäß verwendeten Wirkstoffe hinreichend lagerbeständig sind und keine unerwünschte Umsetzung unter Vernichtung der Epoxidgruppierungen stattfindet. Diese Regel ist insbesondere auch bei der Auswahl eventuell vorliegender Substituenten an den Resten $R_1$ und $R_2$ zu berücksichtigen.

Beispiele für die Reste $R_1$ und/oder $R_2$ — soweit sie nicht Wasserstoff bedeuten — sind die folgenden :

Methyl, Ethyl, Propyl, Butyl, Pentyl, Hexyl, die entsprechenden isomeren Reste wie Isopropyl, Isobutyl, tert. Butyl, Isopentyl, entsprechende ungesättigte, insbesondere olefinisch ungesättigte Reste wie Vinyl, Allyl, Butenyl, Phenyl, Benzyl, Xylyl, Trimethylphenyl, Isopropylphenyl, Naphtyl, Cyclopentyl, Cyclohexyl, die entsprechenden mit 1 bis 3 Alkyl- beziehungsweise Alkenyl-Resten substituierten cycloaliphatischen Reste, wobei die Alkyl- beziehungsweise Alkenyl-Substituenten vorzugsweise 1 bis 4 C-Atome aufweisen.

Geeignete Ausgangsverbindungen für die Herstellung der neuen Diglycidyl-pteridine der allgemeinen Formel I sind beispielsweise Lumazin, Alloxazin, Lumichrom, 6,7-Dimethyl-lumazin, 6-beziehungsweise 7-Phenyl-lumazin und 6,7-Diphenyl-lumazin.

## Beispiele

### 1. Herstellung von 1,3-Diglycidyl-lumazin

Die Mischung aus 9,1 g (50 m Mol) Lumazin-hydrat, 92,5 g (1 Mol) Epichlorhydrin, 0,2 g Tetramethylammoniumbromid und 20 g Molekularsieb 0,4 nm wurden 3 Stunden bei 90 °C gerührt, über Nacht bei Raumtemperatur stehen gelassen, nach Zusatz von 4 g (0,1 Mol) gepulvertem Natriumhydroxid 3 Stunden bei 40 °C geführt und anschließend filtriert. Die Mutterlauge wurde eingedampft und der Rückstand and Kieselgel (Merck) chromatographiert. Eluiert wurde mit Methylenchlorid, das 4 % Methanol enthielt. Nach DC-Kontrolle wurden die Fraktionen mit dem höchsten $R_F$-Wert vereinigt, eingedampft und der Rückstand aus Essigsäureethylester umkristallisiert. Es wurden 3,7 g 1,3-Diglycidyl-lumazin vom Schmelzpunkt 145-147 °C erhalten.

Epoxidzahl : ber. 11,5 ; gef. 10,9

2. Herstellung von 1,3-Diglycidyl-alloxazin

Es wird analog zu 1) gearbeitet, jedoch Alloxazin als Ausgangsmaterial eingesetzt. Als Verfahrensprodukt fällt 1,3-Diglycidyl-alloxazin an : Schmp. 172-173 °C, Epoxidzahl : ber. 9,8 gef. 8,9.

3. Mit den isolierten Wirksubstanzen gemäß 1. und 2. werden die nachfolgenden Versuche durchgeführt nach den Testvorschriften des National Cancer Institutes Bethesda, Maryland 20014, veröffentlicht in « Cancer Chemotherapy reports » Part. 3, September 1972, Vol. 3, No 2.

Die Substanzen wurden als wäßrige 1 %ige Injektionslösungen unmittelbar vor der Applikation frisch hergestellt.

Bei Mäusen wurde gemäß Protokoll 1200 (Seite 91 c) die Tumorart P 388 (Leukämie) i. p. mit $10^6$ Zellen/Maus gesetzt. Die mittlere überlebensdauer der unbehandelten Tiere wird bestimmt.

In weiteren Versuchsgruppen wird den behandelten Tieren der Wirkstoff appliziert. In unterschiedlichen Versuchsreihen werden dabei Einzelgaben verschiedener Konzentration verabreicht. In allen Fällen wird eine signifikante Verlängerung der Lebensdauer der behandelten Testtiere gegenüber der mittleren Überlebensdelten Testtiere gegenüber der mittleren Überlebensdauer der nicht mit dem Wirkstoff behandelten Tiere erzielt. Die Lebensdauer ausgedrückt als T/C in Abhängigkeit von der Dosierung des Wirkstoffes ist wie folgt :

| Verabreichte Dosis mg/kg | T/C-Wert (%) 1) | 2) |
|---|---|---|
| 100 | | 292 (3c)$^+$ |
| 50 | 250 (2c)$^+$ | 155 |
| 25 | 130 | 156 |
| 12,5 | 120 | 125 |

3 bzw. 2 von 6 Mäusen überlebten den Beobachtungszeitraum von 30 Tagen.

**Patentansprüche**

1. Glycidyl-substituierte Pteridin-Verbindungen der allgemeinen Formel I

(I)

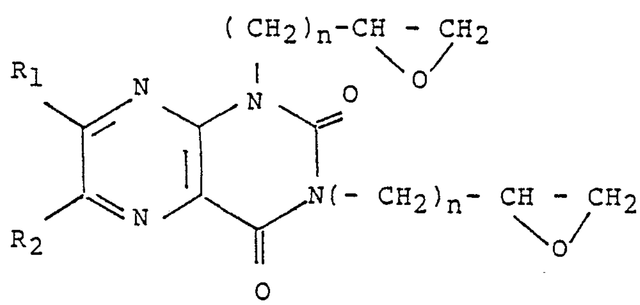

in der $R_1$ und $R_2$ gleich oder verschieden sind und Wasserstoff oder Kohlenwasserstoffreste mit bis zu 12 C-Atomen, die auch N, O, S und/oder P als Heteroatome enthalten können und n die Zahl 1 oder 2 bedeuten, wobei $R_1$ und $R_2$ auch gemeinsam zu einem carbocyclischen oder heterocyclischen Ring geschlossen sein können, der gewünschtenfalls einen Halogen-, Alkoxy-, Cyano-, Hydroxyl-, N-substituierten Amino-, Alkylsulfoxy-, Arylsulfoxy-, Aroxy- oder Acyloxysubstituenten enthalten kann.

2. Glycidyl-substituierte Pteridin-Verbindungen nach Anspruch 1, dadurch gekennzeichnet, daß die Reste $R_1$ und/oder $R_2$ bei ihrer Bedeutung als Kohlenwasserstoffreste nicht mehr als 8 insbesondere nicht mehr als 6 Kohlenstoffatome aufweisen, wobei die Summe aller Kohlenstoffatome in diesen Resten die angegebenen Grenzwerte nicht übersteigt.

3. Verfahren zur Herstellung der neuen Glycidyl-substituierten Pteridin-Verbindungen der allgemeinen Formel I, nach Anspruch 1, dadurch gekennzeichnet, daß man die 1,3-N-unsubstituierten Ausgangsverbindungen der allgemeinen Formel II

(II)

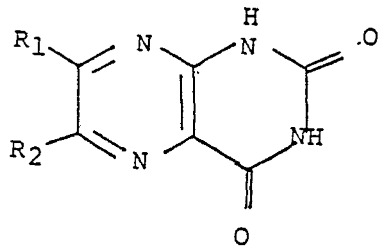

in an sich bekannter Weise, mit Epihalohydrinen umsetzt und nachfolgend Halogenwasserstoff mittels Alkali abspaltet.

4. Arsneimittelzubereitungen mit cytostatischer Wirksamkeit enthaltend Glycidyl-substituierte Pteridin-Verbindungen der allgemeinen Formel I nach Anspruch 1, vorzugsweise in Abmischung mit üblichen Hilfs- beziehungsweise Trägerstoffen.

## Claims

1. New glycidyl-substituted pteridine compounds corresponding to the following general formula

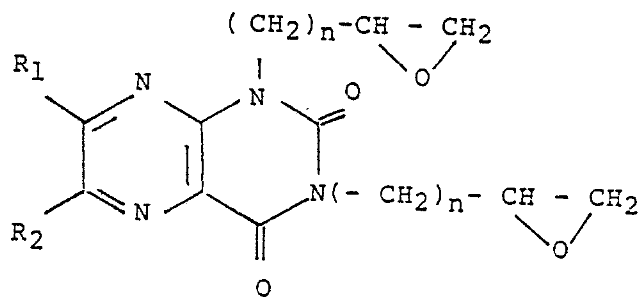

in which $R_1$ and $R_2$ may be the same or different and represent hydrogen or hydrocarbon radicals containing up to 12 carbon atoms, which may also contain N, O, S and/or P as heteroatoms, and n is the number 1 or 2, in addition to which $R_1$ and $R_2$ may be closed to form a carbocyclic or heterocyclic ring which, if desired, may contain a halogen, alkoxy, cyano, hydroxyl, N-substituted amino, alkyl sulfoxy, arylsulfoxy, aroxy or acyloxy substituent.

2. New glycidyl-substituted pteridine compounds as claimed in Claim 1, characterized in that, where they represent hydrocarbon radicals, $R_1$ and/or $R_2$ contain no more than B and, more particularly, no more than 6 carbon atoms, the sum of all the carbon atoms in these radicals not exceeding the limits indicated.

3. A process for producing the new glycidyl-substituted pteridine compounds corresponding to general formula I, characterized in that the 1,3-N-unsubstituted starting compounds corresponding to the following general formula

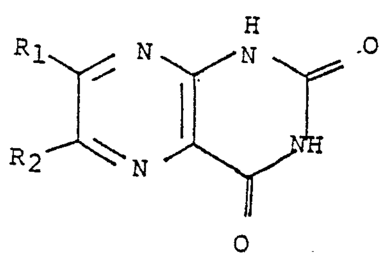

are obtained in known manner, more particularly by reaction with epihalohydrins and subsequent dehydrohalogenation with alkali

4. Cytostatically active medicinal preparations containing glycidyl-substituted pteridine compounds corresponding to general formula I, preferably in admixture with standard auxiliaries and carriers.

## Revendications

1. Nouveaux composés de ptéridine glycidyl-substitués de formule générale I

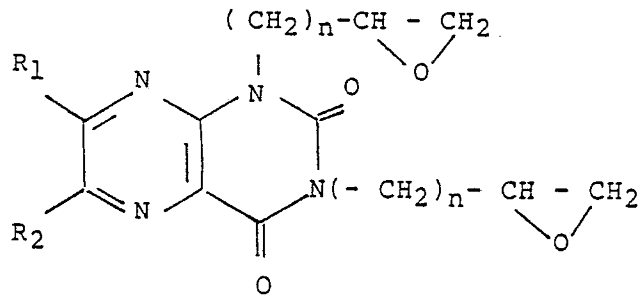

dans laquelle $R_1$ et $R_2$ sont identiques ou différents et signifient de l'hydrogène ou des radicaux hydrocarbonés ayant jusqu'à 12 atomes de carbone, qui peuvent également contenir N, O, S et/ou P en tant qu'hétéroatomes, n'étant le nombre 1 ou 2, ici $R_1$ et $R_2$ pouvant aussi être cyclisés entre eux en un noyau carbocyclique ou hétérocyclique, lequel, si on le désire, peut contenir un substituant halogène, alcoxy, cyano, hydroxyle, amino N-substitué, alcoylsulfoxy, aryl-sulfoxy, aroxy ou acyloxy.

2. Nouveaux composés de ptéridine glycidyl-substitués selon la revendication 1, caractérisés en ce que les radicaux $R_1$ et/ou $R_2$, dans leur signification comme radicaux hydrocarbonés, ne présentent pas plus de 8, en particulier pas plus de 6 atomes de carbone, la somme de tous les atomes de carbone dans ces radicaux ne dépassant pas les valeurs limites indiquées.

3. Procédé de préparation des nouveaux composés de ptéridine glycidyl-substitués de formule générale I, caractérisé en ce qu'on obtient les composés de départ 1,3-N-non substitués, de formule générale II

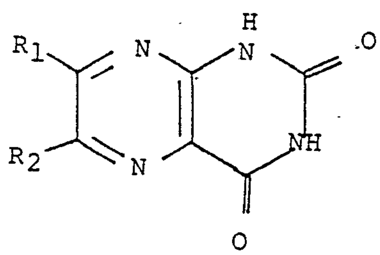

d'une manière connue en elle-même, en particulier par réaction avec des épihalohydrines et clivage consécutif d'hydracide halogéné au moyen d'un alcali.

4. Préparations médicamentaires à activité cytostatique contenant des composés de ptéridine glycidyl-substitués de formule générale I, de préférence en mélange avec des substances auxiliaires ou de support usuelles.